(19) Europäisches Patentamt
European Patent Office
Office européen des brevets

(11) Numéro de publication: **0 072 268 B1**

# (12) FASCICULE DE BREVET EUROPEEN

(45) Date de publication du fascicule du brevet: **16.10.85**

(51) Int. Cl.⁴: **C 07 J 31/00**, A 61 K 31/57

(21) Numéro de dépôt: **82401225.6**

(22) Date de dépôt: **30.06.82**

(54) **Stéroides estérifiés en la position 17 et thioestérifiés en la position 21, leur procédé de préparation et leur application comme médicament.**

(30) Priorité: **30.07.81 FR 8114860**

(43) Date de publication de la demande: **16.02.83 Bulletin 83/7**

(45) Mention de la délivrance du brevet: **16.10.85 Bulletin 85/42**

(84) Etats contractants désignés: **AT DE LU NL SE**

(56) Documents cités:
**FR - A - 2 070 077**
**FR - A - 2 081 395**
**FR - A - 2 231 374**
**FR - A - 2 442 856**

**Le dossier contient des informations techniques présentées postérieurement au dépôt de la demande et ne figurant pas dans le présent fascicule.**

(73) Titulaire: **JOUVEINAL S.A., Tour Maine Montparnasse 33 Avenue du Maine, F-75755 Paris Cedex 15 (FR)**

(72) Inventeur: **Torossian, Diéran Robert, 11, rue Jean Bastard, F-92340 Bourg-La-Reine (FR)**
Inventeur: **Aubard, Gilbert Gustave, 7, Chemin de la Savetière, F-91120 Palaiseau (FR)**
Inventeur: **Grouhel, Agnès Geneviève, 2, rue des Peupliers, F-92190 Meudon (FR)**
Inventeur: **Roux, Claude Paul Joseph, 32, Square Montsouris, F-75014 Paris (FR)**

(74) Mandataire: **Bourgognon, Jean-Marie et al, Cabinet Flechner 22, Avenue de Friedland, F-75008 Paris (FR)**

**EP 0 072 268 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne des stéroïdes nouveaux, leur procédé de préparation et leur applictation en médecine.

Un des chefs de file des stéroïdes anti-inflammatoires locaux est le 17.21 dipropionate de bêtaméthasone. Il s'avère que dans les tests pharmacologiques classiques, la majorité des produits de la présente invention sont plus actifs et ce, pour certains, plus de 100 fois.

Les composés suivant l'invention répondent à la formule:

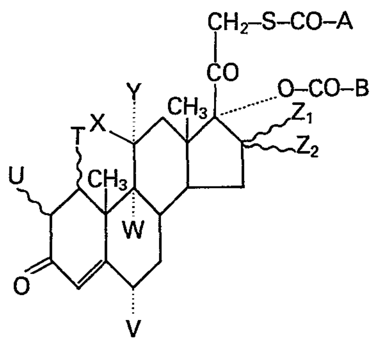

dans laquelle A et B sont chacun, indépendamment l'un de l'autre, un radical alcoyle linéaire ou ramifié ayant de 1 à 6 atomes de carbone ou un radical phényle éventuellement mono- ou plurisubstitué par de radicaux alcoyle ayant de 1 à 6 atomes de carbone, alcoxy ayant de 1 à 6 atomes de carbone ou halogène, T et U sont indépendamment l'un de l'autre des atomes d'hydrogène ou forment ensemble une double liaison, V est un atome d'hydrogène ou un radical méthyle en position α, W est un atome d'hydrogène ou un atome d'halogène en position α, X est un radical hydroxy en position β et Y est un atome d'hydrogène ou X et Y peuvent représenter ensemble un atome d'oxygène, et $Z_1$ est un atome d'hydrogène, un radical méthyle en position α ou β alors que $Z_2$ est un atome d'hydrogène ou $Z_1$ et $Z_2$ forment ensemble un radical méthylène.

Ces composés distinguent de ceux décrits au brevet français No 2081395 en ce qu'il ne sont pas floures en la position 6. Bien que le brevet français No 2081395 ne donne pas de renseignements chiffrés sur l'activité des composés qu'il revendique, ces derniers, qui n'ont pas remplacé le 17, 21 dipropionate de bêtaméthasone sur le marché, ne peuvent qu'être moins intéressants que celui-ci.

Le brevet FR-A-2070077 englobe dans la formule générale 1 page 4 les composés suivant l'invention, sans citer des composés esters en 17 bien spécifiques. Or, ces composés bien spécifiques se révèlent avoir une activité pharmacologique très remarquable.

La préparation des produits de l'invention consiste à faire réagir un 21 sulfonate de stéroïde de formule:

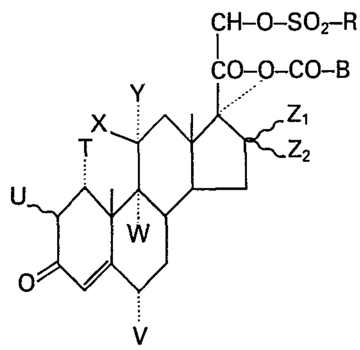

dans laquelle R correspond à un radical alcoyle inférieur ($C_1$ à $C_{12}$, notamment $C_1$ à $C_4$), avec un thiocarboxylate de métal alcalin de formule:

M–S–CO–A

dans laquelle M représente un atome de métal alcalin.

Les 21 sulfonates sont connus en soi et leur préparation réalisée par différentes méthodes est déjà déscrite.

La méthode généralement employé consiste à partir d'un stéroïde 17α,21 dihydroxylé:

1) à estérifier la structure en 17α hydroxy, ce qui se réalise d'une façon bien connue avec un tri-(alcoyl) inférieur)orthocarboxylate approprié dans un solvant organique inerte comme le diméthylsulfoxyde ou le diméthylformamide et en présence d'un catalyseur acide, comme l'acide p-toluènesulfonique.

Le 17,21-orthoester formé est hydrolyse sélectivement en 21 ou moyen d'un acide de force modérée comme l'acide acétique, pour conduire au dérivé 17α-ester-21-hydroxyle.

2) A préparer l'ester sulfonique en 21 du dérivé précédent, ce qui se réalise par des procédés connus en soi, par exemple par action d'un alkylsulfochlorure dans la pyridine.

La préparation des produits de l'invention consiste à faire réagir le 21-sulfonate de formule (II) avec le thiocarboxylate alcalin de formule (III) dans un solvant aprotique, dans des conditions appropriées. Le produit brut obtenu est purifié généralement par chromatographie sur colonne, puis par recristallisation dans un alcool, notamment un alcanol de bas poids moléculaire, pour obtenir un produit d'une pureté satisfaisante.

Dans un premier temps, on effectue la préparation du thiocarboxylate alcalin par salification d'un acide S-thiocarboxylique avec un alcoolate alcalin dans un solvant aprotique, tel qu'une cétone, un amide.

Cette réaction s'effectue en adjoutant une quantité stoechiométrique ou inférieure à la stoechiométrie de 20% d'une solution méthanolique d'environ 4 N de méthylate de sodium sur une solution de 10 à 50% en poids de l'acide S-thiocarboxylique dans l'acétone ou dans l-hexaméthylphosphorotriamide (dite ci-après H.M.P.T.). Le mélange est agité pendant une période de 10 min à 3 h, mais plus favorablement durant une période de 15 à 45 min.

En solution dans le même solvant que précédemment le 21-sulfonate de formule (II), est introduit dans le mélange précédent. Ce produit est engagé en défaut stoechiométrique par rapport à l'acide S-thiocarboxylique. On engage 0,2 à 0,9 mole de (II) par rapport à une mole d'acide, de préférence de 0,5 à 0,85 mole.

Selon le solvant utilisé et les réactifs mis en œuvre la réaction est ensuite effectuée à une température comprise entre 0 et 100°C environ durant une période qui varie de 5 min à 10 h. De préférence les températures réactionnelles sont comprises entre 20 et 60°C et les durées entre 10 min et 8 h.

Le produit formé est ensuite isolé par différentes méthodes faisant appel à la distillation ou à l'extraction par des solvants. La purification est généralement effectuée par chromatographie sur colonne suivie d'une recristallisation finale dans un alcool ou un mélange alcool-eau. Les alcools préférés sont le méthanol et l'éthanol, pour les mélanges alcool-eau, celui formé par 80% de méthanol et 20% d'eau est préféré.

L'invention vise enfin un médicament incorporant un composé suivant l'invention à titre de principe actif.

Les exemples suivants illustrent l'invention. Dans ces exemples:

a) Le degré de pureté des produits obtenus a été suivi par chromatographie sur couches minces:

support: gel de silice 60 F 254 (fournisseur Merck),

dépôts: 100 mcg de produit,

rélévation: observation des plaques sous lumière ultra-violette de longueur d'onde de 254 nm.

b) Les déterminations des points de fusion ont été réalisées sur un appareil «Mettler FP 1» et ne sont pas corrigées.

c) Les analyses élémentaires des produits ont été réalisées; elles ne sont pas rapportées dans les exemples. Elles sont en bon accord avec la théorie. Il en est de même pour les spectres d'absorption infrarouge, qui ont été enregistrés par la méthode de la pastille en suspension dans le KBr. Les principales absorptions, compatibles avec les structures décrites, ne sont pas présentées.

d) Les spectres de résonance magnétique nucléaire du proton sont présentés. Ils ont été enregistrés en solution dans le deutérochloroforme $CDCl_3$ sur un appareil de 60 MHz.

Les déplacements chimiques sont exprimés en p.p.m. par rapport au tétraméthylsilane pris comme référence.

L'aspect des signaux est présenté ainsi que l'attribution de leur position sur la structure:

s = singulet
d = doublet
t = triplet
q = quadruplet
m = massif
J = constantes de couplage en $H_z$.

Exemple: 0,98 (s – 18 $CH_3$) est le signal en singulet qui correspond aux trois hydrogènes portés par le carbone 18.

Note: Le «Florisil» est la dénomination commerciale d'un support pour chromatographie (Floridin Company, USA).

Exemple 1

21-thiovalérate,17-valérate de 3,20-dione-11β-hydroxyprègn-4-ène (ou d'hydrocortisone).

A et B = butyle normal T, U, V, W, Y, Z = H, X, = OH.

Dans un réacteur, on introduit 28,3 grammes (0,24 mole) d'acide S-thiovalérique et 900 ml d'acétone.

Sous agitation, à une température d'environ 20°C, on introduit 67 ml d'une solution méthanolique de méthylate de sodium 3,58 N (0,24 mole).

Après une heure d'agitation à la température ambiante, on ajoute 90,0 g (0,17 mole) de 21-mésylate,17-valérate de cortisol en solution dans 1800 ml d'acétone.

La suspension obtenue est portée au reflux de l'acétone (56,2°C) sous agitation durant 4 h.

L'acétone est éliminée par distillation jusqu'à obtention d'un volume résiduel d'un litre environ.

Le résidu est précipité dans deux litres d'eau froide; la gomme qui se forme est extraite à l'éther diéthylique.

Les phases éthérées réunies sont évaporées. Poids de résidu 94 g.

Le résidu est purifié par chromatographie sur colonne à l'aide de 3,6 kg de «Florisil» (60 – granulométrie inférieure à 0,15 mm). L'élution, d'abord par un mélange benzène-acétone 98–2 (v/v), puis par un mélange benzène-acétone 95–5 (v/v) permet de recueillir avec ce dernier solvant 42,5 grammes de produit purifié.

Ce produit est finalement recristallisé dans 1200 ml de mélange méthanol-eau 8–2 (v/v).

Poids = 16,6 g

Rendement = 17,7%

Point de fusion = 131°C

R.M.N. = 0,93 (t, $CH_3$ esters); 0,98 (s, 18 $CH_3$) 1,45 (s, 19 $CH_3$); 1,75 (m, 11 BOH); 2,35 (q, $CH_2$ esters); 3,80 (s, 21 $CH_2$); 4,5 (m, 11 CH); 5,70 (s, 4 CH).

Exemple 2

21-thiopivalate,17-valérate de 3,20-dione-11β hydroxy-prègn-4-ène (ou d'hydrocortisone).

A = tertiobutyle; B = n-butyle; T, U, V, W, Y, Z = H; X = OH.

De la même manière que dans l'exemple 1, à partir de 60,0 grammes (0,114 mole) de 21-mésylate, 17-valérate de cortisol, de 19,0 grammes (0,160 mole) d'acide S-thiopivalique et de 45,6 ml (0,160 mole) d'une solution méthanolique de méthylate de sodium 3,5 N, on obtient un produit qui est finalement purifié par recristallisation dans un mélange méthanol-eau 80–20 (v.v).

Poids = 18,0 g

Rendement = 28,8%

Point de fusion = 206°C

R.M.N. = 0,92 (t, $CH_3$ esters); 0,96 (s, 18 $CH_3$); 1,27 (s, t-butyle); 1,45 (s, 19 $CH_3$); 1,70 (m, 11 βOH; 2,01 (q, $CH_2$ ester); 3,79 (s, 21 $CH_2$); 4,50 (m, 11 CH); 5,70 (s, 4 CH).

Exemple 3

21-thioacétate,17-butyrate de 3,11,20-trione-prègnadiène-1,4 (ou de prednisone).

A = méthyle; B = n-propyle; T et U = O (double liaison); V, W, Z = H; X et Y = fonction céto.

Dans un réacteur d'un litre, on introduit 290 ml d'hexaméthylphosphorotriamide (H.M.P.T.) et 8,7 g d'acide S-thioacétique (0,115 mole). On ajoute 25,2 ml d'une solution méthanolique de méthylate de sodium 4,5 N (0,115 mole).

Le mélange est agité 30 min à la température ordinaire, puis on y ajoute 29,1 g (0,0574 mole) de 21-mésylate 17-butyrate de prednisone en solution dans 580 ml d'H.M.P.T.

Après 10 min d'agitation à la température ambiante, le milieu réactionnel est précipité dans l'eau glacée et le mélange est extrait à l'éther diéthylique.

Le résidue des phases éthérées représente 26,0 g d'un mélange de produits qui est purifié par chromatographie sur une colonne de 500 g de «Florisil».

L'élution par un mélange hexane-acétone permet d'obtenir 13,7 g de produit finalement purifiés par recristallisation dans un mélange méthanol-eau 80–20 (v/v).

Poids = 9,6 g

Rendement = 34,4%

Point de fusion = 114,5°C

R.M.N. = 0,72 (s, 18 $CH_3$); 0,98 (t, $CH_3$ ester en 17); 1,45 (s, 19-$CH_3$); 2,35 (t, $CH_2$ ester); 2,37 (s, ester en 21); 3,80 (s, 21-$CH_2$); 6,19 (s, 4-CH); 6,37 (d, J = 2 Hz, 2-CH); 7,75 (D,J = 10 Hz, 1-CH).

Exemple 4

21-thioacétate-17 benzoate de 3,20-dione-11β hydroxy 6α méthyl-prègna-diène-1,4 (ou de la 6α méthylprédnisolone).

A = méthyle; B = phényle; T et U = O (double liaison); V = méthyle en position α; W, Y et Z = H; X = OH.

Sur agitation électromagnétique, en prépare une solution de 1,765 ml d'acide S-thioacétique (24,8 mmoles) dans 165 ml d'HMPT.

On ajoute ensuite 5,865 ml d'une solution méthanolique de méthylate de sodium 4 N (23,5 mmoles).

Après une heure d'agitation, on introduit 8,2 g (15,6 mmoles) de 21-mésylate-17-benzoate de 6α méthyl-prédnisolone.

Le mélange réactionnel est agité pendant 6 h à température ambiante, puis précipité dans l'eau.

Le mélange est extrait à l'éther diéthylique. Le résidu de la phase éthérée est purifié par chromatographie sur une colonne de 300 g de «Florisil».

On obtient 4,5 g de produit purifié qui finalement est recristallisé dans le méthanol.

Poids = 3,1 g

Rendement = 37,0%

Point de fusion = 215°C (décomposition)

R.M.N = 1,05 (s, 18, $CH_3$); 1,15 (d, 6-$CH_3$); 1,52 (s, 19-$CH_3$); 1,95 (m, 11β OH); 2,35 (s, $CH_3$ ester en 21); 4,60 (m, 11-CH); 7,65 (m-noyau phényle).

En procédent comme dans l'exemple 4 avec les réactifs appropriés, on prépare les produits suivants:

Exemple 5

21-thiopropionate-17-propionate de 3,20-dione-11β-hydroxy 6α-méthyl-prègna-diène-1,4 (ou de 6α méthyl-prédnisolone).

A et B = éthyle; T et U = O (double liaison); V = méthyle en position α; W, Y et Z = H; X = OH

Rendement = 11%

Point de fusion = 161°C

R.M.N. = 1,00 (s, 18-$CH_3$); 1,10 (d, 6-$CH_3$); 1,20 (s, 19-$CH_3$); 1,47 (s, esters propioniques); 1,68 (m, 11 βOH); 3,85 (s, 21-$CH_2$); 6,08 (s, 4-CH).

Exemple 6

21-thiovalérate-17 valérate de 3,20-dione-11β hydroxy 6α-méthyl-prègna-diène-1,4 (ou de 6α méthylprédnisolone).

A et B = butyle; T et U = O (double liaison); V = méthyle en position α; W, Y et Z = H; X = OH.

Rendement = 47,4%

Point de fusion = 170°C

R.M.N. = 0,98 (s, 18-$CH_3$); 1,15 (d, 6-$CH_3$); 1,47 (s, 19-$CH_3$); 1,95 (m, 11 βOH); 3,80 (s, 21-$CH_2$); 6,07 (s, 4-CH).

Exemple 7

21-thioacétate-17 valérate de 3,20-dione-11β hydroxy 6α méthyl-prègn-diène 1,4 (ou de 6α méthylprédnisolone).

A = méthyle; B = butyle; T et U = O (double liaison) V = méthyle en position α; W, Y et Z = H; X = OH.

Rendement = 27%

Point de fusion = 146,5°C

R.M.N. = 1,00 (s, 18-$CH_3$); 1,12 (d, 6-$CH_3$); 1,47 (s, 19-$CH_3$); 2,02 (m, 11 βOH); 3,82 (s, 21-$CH_2$); 6,02 (s, 4-CH).

Exemple 8

21-thioacétate,17-acétate de 3,20-dione-9α-fluoro-11β-hydroxy-16β-méthyl-prègna-diène-1,4 (ou de bêta-méthasone).

A = B = méthyle; T et U = O (double liaison); V et Y = H; X = βOH; Z = βméthyle.

Dans un réacteur pouvant supporter une faible pression, on introduit 500 ml d'acétone et 8,83 g (116 mmoles) d'acide S-thioacétique.

Sous agitation, on introduit, en 5 min, à la température ambiante, 25,8 ml d'une solution méthanolique de méthylate de sodium 4,5 N (116 mmoles).

Après avoir laissé agiter le mélange à la température ambiante pendant 45 min, on introduit 49,5 g (96,6 mmoles) de 21-mésylate-17 acétate de bêtaméthasone en solution dans 900 ml d'acétone.

Le réacteur est hermétiquement clos et est porté à 56°C ± 2°C pendant 5 h. Durant cette période le contenu de réacteur est agité de temps à autre.

Après refroidissement, on élimine par distillation environ 700 ml de solvants. Le résidue est

précipité dans l'eau glacée et le mélange est extrait à l'éther diéthylique.

Les 49 g de résidu obtenus à partir de cette extraction éthérée sont purifiés par une chromatographie sur une colonne contenant 1,4 kg de «Florisil». Ce traitement par élution avec un mélange benzène-acétone permet d'obtenir 12,3 g de produit qui subissent une dernière purification par recristallisation dans 140 ml d'éthanol.

Poids = 8,4 g

Rendement = 17,65%

Point de fusion = 207°C

R.M.N. = 0,95 (s, 18-$CH_3$); 1,32 (d, J = 6 Hz, 16β-$CH_3$); 2,13 (s, méthyl ester C-17); 2,40 (s, méthyl thioester, 0,21); 3,13 (m, 11β OH); 6,15 (s, 4-CH); 6,35 (d-d, J1 = 10 Hz, J2 = 3 Hz, 2-CH); 7,30 (d, J = 10 Hz, 1-CH).

En procédant comme à l'exemple 8, avec les réactifs appropriés, on prépare les produits suivants.

Exemple 9

21-thiopropionate 17-acétate de 3,20-dione-9α-fluoro-11β-hydroxy-16β-méthyl-prègna-diène-1,4 (ou de béta-méthasone).

A = éthyle; B = méthyle; T et U = O (double liaison) V et Y = H; X = βOH; Z = βméthyle.

Rendement = 13,3%

Point de fusion = 193°C

R.M.N. = 0,93 (s, 18-$CH_3$); 1,17 (t, $CH_3$ ester 21); 1,35 (d, J = 6 Hz, 16β-$CH_3$); 1,55 (s, 19-$CH_3$); 2,09 (s, méthyl, ester C-17); 2,20 (m, 11β ON); 3,55 et 3,77 (d-d, J = 7, 21-$CH_2$); 6,15 (s, 4-CH); 7,25 (d, J = 10 Hz, 1-CH).

Exemple 10

21-thioacétate 17-propionate de 3,20-dione-9α-fluoro-11β-hydroxy-16β-méthyl-prègna-diène-1,4 (ou de bêta-méthasone).

A = méthyle; B = éthyle; T et U = O (double liaison) V et Y = H; X = βOH; Z = βméthyle.

Rendement = 26,75%

Point de fusion = 187,5°C

R.M.N = 0,95 (s, 18-$CH_3$); 1,10 (t, méthyl ester C–17); 1,36 (d, 16-CH); 2,32 (s, méthyl ester 21); 3,40 (m, 11β OH); 3,68 (s, 21-$CH_2$); 7,35 (d, J = 10 Hz 1-CH).

Exemple 11

21-thiopropionate 17-propionate de 3,20-dione-9α-fluoro-11β hydroxy-16β méthyl-prègna-diène-1,4 (ou de bêta-methasone).

A = B = éthyle; T et U = O (double liaison); V et Y = H; X = βOH; Z = βméthyle.

Rendement = 31,2%

Point de fusion = 175°C

R.M.N. = 0,96 (s, 18-$CH_3$); 1,15 (t, $CH_3$ esters); 1,35 (d, 16-CH); 1,55 (s, 19-$CH_3$); 2,80 (m, 11β OH); 3,50 et 3,82 (d, d, J = 17 Hz, 21-$CH_2$).

Exemple 12

21-thiovalérate 17-valérate de 3,20-dione-9α-fluoro-11β hydroxyx-16β méthyl-prègna-diène,1,4 (ou de bêta-méthasone).

A = B = butyle; T et U = O (double liaison); V et Y = H; X = βOH; Z = βméthyle.

Rendement = 42,8%

Point de fusion 148°C

R.M.N. = 0,93 (t, $CH_3$ esters); 0,96 (s, 18-$CH_3$); 1,35 (d, J = 6 Hz, 16β-$CH_3$); 1,55 (s, 19-$CH_3$); 2,18 (m, 11-OH); 3,50–3,90 (d,d, J = 17 Hz, 21-$CH_2$); 7,25 (d, J = 10 Hz, 1CH).

Exemple 13

21-thioacétate 17-valérate de 3,20-dione-9α-fluoro 11β-hydroxy-16β méthyl-prègna-diène-1,4 (ou de bêta-méthasone).

A = méthyle; B = butyle; T et U = O (double liaison); V et Y = H; X = βOH; Z = βméthyle.

Rendement = 37%

Point de fusion = 127,5°C

R.M.N. = 0,97 (s, 18-$CH_3$); 1,32 (d, 16-$CH_3$β); 1,57 (s, 19-$CH_3$); 2,77 (m, 11-βOH); 6,15 (s, 4-CH).

Exemple 14

21-thiotertiobutylacétate 17-acétate de 3,20-dione-9α-fluoro-11β-hydroxy-16α-méthyl-prègna-diène-1,4 (ou de dexaméthasone).

A = 2,2-diméthyl-n-propyle; B = méthyle; T et U = O (double liaison); V et Y = H; W = fluor; X = βOH; Z = αméthyle.

Dans un réacteur on introduit 360 ml d'HMPT et 18,5 grammes (0,140 mole) d'acide S-thiotertiobutylacétique.

A la température ambiante on ajoute 31,1 ml de solution méthanolique de méthylate de sodium 4,5 N (0,140 mole).

Après une heure d'agitation on introduit en 15 min environ 36,0 g (0,070 mole) de 21 mésylate 17-acétate de dexaméthasone en solution dans 720 ml d'HMPT.

Le milieu réactionnel est agité pendant 24 h à la température ordinaire, précipité dans l'eau et extrait à l'éther. Le résidu de la phase éthérée est purifié par trois recristallisations successives dans un mélange méthanol-eau 80.20 (v/v).

Poids = 15,5 g

Rendement = 40,3%

Point de fusion = 149°C

R.M.N. = 0,91 (d, 16α-$CH_3$); 1,02 (s, 18-$CH_3$); 1,05 (s, +.butyl ester 21); 1,55 (s, 19-$CH_3$); 2,10 (s, $CH_3$ ester 17); 3,48 (s, 11β-OH); 7,25 (d, J = 10 Hz, 1-CH).

Exemple 15

21-thiopropionate 17-propionate de 3,20-dione-9α chloro-11β hydroxy-16β méthyl-prègna-diène-1,4 (ou de béclométhasone).

A = B = éthyle; T et U = O (double liaison); V et Y = H; W = αchloro; X = βOH; Z = βméthyle.

Dans un réacteur pouvant être fermé hermétiquement et pouvant résister à une légère pression, on introduit 150 ml d'acétone et 3,9 g (43,2 mmoles) d'acide S-thiopropionique).

On ajoute ensuite 9,55 ml d'une solution méthanolique de méthylate de sodium 4,5 N (43,2 mmoles). Le mélange est agité à la température ambiante pendant 45 min, puis on introduit 13,0 g

(24 mmoles) de 21-mésylate 17-propionate de béclométhasone en solution dans 260 ml d'acétone.

Le mélange est agité, puis le réacteur hermétiquement clos est porté pendant 19 h dans une étuve régulée à 60°C. Au cours du temps, le mélange se colore progressivement pour finalement devenir noir.

Après refroidissement, le milieu réactionnel est précipité dans 3 l d'eau froide et extrait à l'éther diéthylique.

Le résidu obtenu à partir des phases éthérées pèce 13,7 g et se présente sous forme d'une gomme verdâtre. Il est purifié par chromatographie sur une colonne de 430 grammes de «Florisil».

L'élution par un mélange chlorure de méthylène-acétone 98–2 (v/v) permet d'obtenir 7,5 g de produit purifié qui subissent une dernière recristallisation dans 60 ml de mélange méthanol-eau 80–20 (v/v).

Poids = 4,0 g

Rendement = 30%

Point de fusion = 160°C

R.M.N. = 0,99 (s, 18-$CH_3$); 1,18 (t, $CH_3$ esters); 1,37 (d, 16β-$CH_3$); 1,68 (s, 19-$CH_3$); 1,80 (m, 11β-OH); 3,50, 3,85 (d.d, J = 17 Hz, 21-$CH_2$); 7,25 (d, J = 10 Hz, 1-CH).

Selon le procédé de l'exemple 15, avec les réactifs appropriés, on prépare les composés suivants:

Exemple 16

21-thiovalérate 17-propionage de 3,20-dione-9α-chloro-11β hydroxy-16β méthyl-prègna-diène-1,4 (ou de béclométhasone).

A = butyle; B = éthyle; T et U = O (double liaison); V et Y = H; W = αchloro; X = βOH; Z = βméthyle.

Rendement = 19%

Point de fusion = 136°C

R.M.N. = 1,00 (s, 18-$CH_3$); 1,37 (d, 16-$CH_3$β); 1,70 (s, 19-$CH_3$); 2,38 (m, 11-βOH); 6,12 (s, 4-CH).

Exemple 17

21-thioacétate 17-valérate de 3,20-dione-9α chloro-11β hydroxy-16β méthyl-prègna-diène-1,4 (ou de béclométhasone).

A = méthyle; B = butyle; T et U = O (double liaison); V et Y = H; W = αchloro; X = βOH; Z = βméthyle.

Rendement = 55,8%

Point de fusion = 138,5°C

R.M.N. = 1,00 (s, 18-$CH_3$); 1,37 (d, 16-β$CH_3$); 2,97 (m, 1-βOH); 6,12 (s, 4-CH).

Exemple 18

21-thiovalérate 17-valérate de 3,20-dione-9α chloro-11β hydroxy-16β méthyl-prègna-diéne-1,4 (ou de béclométhasone).

A et B = butyle; T et U = O (double liaison); V et Y = H; W = αchloro; X = βOH; Z = βméthyle.

Rendement = 35,4%

Point de fusion = 210,5°C

R.M.N. = 0,98 (s, 18-$CH_3$); 1,38 (d, 16-β$CH_3$); 1,68 (s, 19-$CH_3$); 2,54 (m, 11-βOH); 6,10 (s, 4-CH).

Exemple 19

21-thiopropionate 17-propionate de 3,20-dione 11β-hydroxy-16-méthylène-prègna-diène-1,4 (ou de prednylidène).

A = B = éthyle; T et U = O (double liaison); V, Y et W = H; X = βOH; Z = méthylène.

Dans un petit réacteur, on introduit 123 mg (1,36 mmole) d'acide S-thiopropionique et 4,5 ml d'acétone. On ajoute 0,3 ml de solution méthanolique de méthylate de sodium 4 N (1,36 mmole).

Le mélange est agité une heure à la température ambiante puis on ajoute 460 mg (0,908 mmole) de 21-mésylate 17-propionate de prednylidène en solution dans 12 ml d'acétone.

Le mélange agité est porté 2 h 15 au reflux de l'acétone, est ensuite précipité dans l'eau et extrait à l'éther.

Le résidu contenu dans la phase éthérée pèse 450 mg, il est purifié par chromatographie sur une colonne contenant 21 g de «Florisil».

L'élution par un mélange dichlorométhane-acétone 80–20 (v/v) permet d'obtenir 200 mg de produit purifié, qui est recristallisé dans un mélange méthanol-eau 80–20 (v/v).

Poids = 175 mg

Rendement = 98,5%

Point de fusion = 164°C

R.M.N. = 1,05 (s, 18-$CH_3$)o, 1,20 (t, $CH_3$ esters); 1,46 (s, 19-$CH_3$); 1,90 (m, 11β-OH); 3,95 (s, 21-$CH_2$); 5,55 (d, J = 8 Hz, 16 méthylène); 7,30 (d, J = 10 Hz, 1-CH).

Les composés suivant l'invention administrés à l'animal par des voies compatibles à leurs propriétés d'insolubilité dans l'eau, ne présentant pas de phénomènes de toxicité aiguë.

L'activité pharmacologique des produits décrits dans l'invention a été effectuée chez le rat selon une technique voisine de celle décrite par C.A. Winter et C.C. Porter (J. Am. Pharm. Ass. 1957, 46, 9, pages 515–519).

Le principe du test consists à implanter un pellet de coton dans le tissu sous-cutané dorsal de l'animal. Les conséquences de l'ajout sur le pellet de corticoïdes à étudier peut permettre:

1) Détermination de l'activité anti-inflammatoire locale.

Au milieu du pellet la présence de produits peut inhiber ou supprimer la formation d'une granulome.

2) Détermination de l'activité systémique des produits après application locale.

L'administration des corticoïdes par voie locale peut entraîner entre autres:

a) Une diminution des systèmes de défense de l'organisme dont le reflet le plus sensible est la diminution du poids des thymus des animaux.

b) Une perturbation du métabolism protéique qui provoque une fonte tissulaire d'où une inhibition de la croissance pondérale des animaux.

Ces effets ont été étudiés avec les produits de l'invention en utilisant le mode opératoire suivant.

Mode opératoire

Les animaux sont répartis au hasard en lots homogènes de 10 rats mâles Sprague-Dawley

I.O.P.S. de poids corporel compris entre 100 et 120 g.

Les pellets à implanter ont un poid de 35 à 40 mg et sont préparés à partir de rouleaux de coton dentaire. Avant leur insertion chaque pellet est pesé et imprégné avec la quantité exacte du produit à étudier qui a été dissous dans 0,2 ml de chloroforme. Le chloroforme est éliminé par évaporation pendant 24 h à la température ambiante.

Avant implantation chaque pellet est imprégné d'une solution d'antibiotiques:

Pénicilline G 200.000 U.I./ml

Streptomycine 0,1 g/ml

Les animaux sont anesthésiés à l'éther, on implante alors pour chaque animal deux pellets dans le tissu sous-cutané dorsal, de part et d'autre de la ligne médiane:

du côté gauche le pellet qui a reçu le produit à tester (animal traité) ou le véhicule seul (animal témoin);

du côté droit le pellet qui a reçu le véhicule seul.

Les animaux ont une alimentation normale et sont sacrifiés six jours après l'implantation des pellets.

1) Activité anti-inflammatoire locale

Les pellets entourés de tissu granulomateux sont soigneusement prélevés et pesés, puis séchés jusqu'à poids constant.

L'activité des produits est exprimée en pourcentage d'inhibition par rapport aux valeurs des lots témoins et plus particulièrement la dose efficace 50 de produit à étudier (DE 50) qui entraîne une inhibition de 50% du poids.

2) Détermination de l'effet systémique

a) Effet sur le thymus

Sur les mêmes animaux, les thymus sont prélevés et pesés. Par le même type de calcul on détermine une DE 50 qui correspond à la dose de produit qui entraîne une diminution de 50% du poids des thymus des animaux témoins.

b) Effet sur le gain pondéral

Le gain pondéral sur 6 jours est déterminé sur les animaux avant sacrifice.

L'activité des produits a été exprimée en pourcentage d'inhibition de gain des animaux traités par rapport au gain des animaux témoins.

La DE 30 qui correspond à une inhibition de 30% du gain pondéral a été déterminée.

Resultats

1) Activité anti-inflammatoire locale

Les résultats sont reportés dans le tableau 1.

Une expression de l'activité relative des produits à étudier par rapport au 17,21-dipropionate bêtaméthasone est calculée selon le rapport:

$$RI = \frac{\text{DE 50 DPB}}{\text{DE 50 produit étudié}}$$

Tableau I

| Produit | $DE_{50}$ (mg/pellet) | R1 |
|---|---|---|
| D.P.B. | 1,610 | 1 |
| Ex. 1 | <0,200 | >8 |
| Ex. 2 | 2,140 | 0,75 |
| Ex. 3 | 0,600 | 2,7 |
| Ex. 4 | 0,056 | 29 |
| Ex. 5 | 0,210 | 7,7 |
| Ex. 6 | 0,180 | 8,9 |
| Ex. 7 | 0,800 | 2,0 |
| Ex. 8 | 0,072 | 22 |
| Ex. 9 | 0,100 | 16 |
| Ex. 10 | 0,013 | 124 |
| Ex. 11 | 0,012 | 134 |
| Ex. 12 | <0,0125 | >129 |
| Ex. 13 | 0,050 | 32,2 |
| Ex. 14 | 0,003 | 537 |
| Ex. 15 | <0,005 | >322 |
| Ex. 16 | 0,120 | 13,4 |
| Ex. 17 | 0,180 | 8,9 |
| Ex. 18 | 0,096 | 16,8 |
| Ex. 19 | 1,800 | 0,89 |
| Dipropionate de béclométhasone | > 5 | < 0,2 |

La majorité des produits de la présente invention montrent dans ce test une activité égale ou supérieure au 17,21 dipropionate de bêtaléthasone.

Ceci est particulièrement remarquable pour les produits des exemples 10 à 12 et 14 et 15, qui sont des activités plus de 100 fois supérieures.

La comparaison avec le 17,21 dipropionate de béclométhasone est encore plus flatteuse pour les composés de l'invention.

2) Effets systémiques après application locale

a) Effets sur le poids du thymus.

Dans le tableau 2, qui suit, les résultats obtenus et la comparaison relative par rapport au DPB (R2) sont reportés:

Tableau 2

| Produit | $DE_{50}$ (mg/pellet) | R2 |
|---|---|---|
| D.P.B. | 0,86 | 1 |
| Ex. 1 | nulle à 3,2 | <<0,27 |
| Ex. 2 | nulle à 3,4 | <<0,25 |
| Ex. 3 | >>5 | <<0,17 |
| Ex. 4 | >>5 | <<0,17 |
| Ex. 5 | >>1 | <<0,86 |
| Ex. 6 | >>1 | <<0,86 |
| Ex. 7 | >>1 | <<0,86 |
| Ex. 8 | 1,20 | 0,71 |
| Ex. 9 | 1,70 | 0,51 |
| Ex. 10 | 0,25 | 3,40 |
| Ex. 11 | 0,46 | 1,9 |
| Ex. 12 | >>0,8 | <<1 |
| Ex. 13 | 0,8 | 1,1 |
| Ex. 14 | 0,33 | 2,6 |
| Ex. 15 | 2,4 | 0,36 |

Tableau 2

| Produit | $DE_{50}$ (mg/pellet) | R2 |
|---|---|---|
| Ex. 16 | >>1 | <<0,86 |
| Ex. 17 | ><1 | <<0,86 |
| Ex. 18 | >>1 | <<0,86 |
| Ex. 19 | 7 | 0,12 |
| Dipropionate de béclométhasone | 3,1 | 0,27 |

L'activité sur le poid des thymus provoquée localement par les produits de l'invention comparée au 17,21 dipropionate de bêtaméthasone est pour certains produits égale ou supérieure à cette référence.

On remarque que certains produits (exemples Nos. 1 à 7, 16 à 19 ont une activité nettement plus faible).

b) Effets sur le gain pondéral

Les résultats et l'activité relative R3, calculée de la même façon que précédemment à partir des $DE_{30}$ sont reportés dans le tableau 3.

Tableau 3

| Produit | $DE_{30}$ (mg/pellet) | R3 |
|---|---|---|
| D.P.B. | >>0,8 | 1 |
| Ex. 1 | >>3,2 | <<0,25 |
| Ex. 2 | >>3,4 | <<0,24 |
| Ex. 3 | >5 | <0,16 |
| Ex. 4 | >5 | <0,16 |
| Ex. 5 | >>1 | <<0,8 |
| Ex. 6 | >>1 | <<0,8 |
| Ex. 7 | >>1 | <<0,8 |
| Ex. 8 | 1 | 0,8 |
| Ex. 9 | 1 | 0,8 |
| Ex. 10 | >1 | <0,8 |
| Ex. 11 | 0,8 | 1 |
| Ex. 12 | >>0,8 | <<1 |
| Ex. 13 | 0,65 | 1,23 |
| Ex. 14 | 0,015 | 53 |
| Ex. 15 | 0,56 | 1,42 |
| Ex. 16 | >>1,0 | <<0,8 |
| Ex. 17 | >>1,0 | <<0,8 |
| Ex. 18 | >>1,0 | <<0,8 |
| Ex. 19 | 0,015 | 53 |
| Dipropionate de béclométhasone | 5 | 0,16 |

La majorité des produits de l'invention présentent une activité comparable ou inférieure au 17,21 dipropionate de bêtaméthasone pris comme substance de référence.

En conclusion des tests pharmacologiques effectués, les produits de l'invention présentent une activité anti-inflammatoire locale intéressante, égale et parfois très supérieure à celle du 17,21-dipropionate de bêtaméthasone pris comme substance de référence.

En outre, les résultats des tests effectués pour rechercher les effets systémiques montrent que les produits de l'invention ont pour la plupart une activité inférieure à celle de la référence.

La supériorité des produits de l'invention par rapport à la référence peut être exprimée par le rapport R1/R2 qui tient compte, pour R1, de l'activité anti-inflammatoire locale et, pour R2, de l'activité systémique.

$$R4 = \frac{R1}{R2} = \frac{DE_{50}\ \text{D.P.B (pellet)}}{DE_{50}\ \text{prdts (pellet)}} \times \frac{DE_{50}\ \text{prdts (thymus)}}{DE_{50}\ \text{D.P.B. (thymus)}}$$

En conséquence, plus la valeur de R4 est élevée, plus le produit de l'exemple présente une activité anti-inflammatoire locale favorable par rapport au 17,21-dipropionate de bêtaméthasone (D.P.B.). Les résultats de ces calculs sont présentés dans le tableau 4.

Tableau 4

| Produit | R4 | Produit | R4 |
|---|---|---|---|
| D.P.B. | 1 | Ex.10 | 36 |
| Ex. 1 | 30 | Ex.11 | 71 |
| Ex. 2 | 3 | Ex.12 | 129 |
| Ex. 3 | 16 | Ex.13 | 29 |
| Ex. 4 | 171 | Ex.14 | 207 |
| Ex. 5 | >9 | Ex.15 | 894 |
| Ex. 6 | >10 | Ex.16 | >16 |
| Ex. 7 | >2 | Ex.17 | >10 |
| Ex. 8 | 31 | Ex.18 | >20 |
| Ex. 9 | 31 | Ex.19 | 7 |
| Dipropionate de béclométhasone 0,7 | | | |

Ces résultats démontrent les propriétés anti-inflammatoires locales intéressantes des composés de l'invention.

On préfère tout particulièrement les composés des exemples 4, 11, 12, 14 et 15.

Les produits de l'invention peuvent être administrés à l'homme par les voies appropriées aux différents sites des affections inflammatoires sous forme de compositions pharmaceutiques adaptées à l'administration par voie topique et/ou systémique.

La quantité de stéroïde actif dans ces compositions pharmaceutiques dépend de l'activité anti-inflammatoire intrinsèque de ce stéroïde et de la nature de l'affection inflammatoire à traiter.

Les compositions administrées par voie topique peuvent contenir avantageusement l'ingrédient actif en des quantités comprises entre 0,01 et 5% en poids en mélange avec un excipient classique. Ces compositions peuvent être appliquées une à plusieurs fois par jour selon la nature et la gravité de l'affection à traiter.

Pour l'administration systémique, les compositions retenues peuvent contenir selon la nature de l'agent actif de 0,1 à 100 mg du produit actif par unité de dosage de 0,1 à 100 g. Journellement la quantité de principe actif administrée peut varier de 0,1 à 300 mg.

Il est entendu que les compositions pharmaceutiques peuvent contenir d'autres principes actifs. A titre d'exemples non limitatifs des substances telles que des agents de conservation, des agents bactériostatiques, des antibiotiques, des agents antimycotiques et des anesthésiques locaux.

Pour l'application topique des agents actifs sont incorporés à des excipients ou des supports conventionnellement employés pour la préparation de compositions pharmaceutiques appropriées à cette administration. Ces compositions peuvent être par exemple des onguents, des lotions, des crèmes, des émulsions, des gouttes, des lavements, des suppositoires, des ovules, des instillations, des aérosols.

Les compositions pharmaceutiques adaptées à l'administration systémique peuvent être liquides ou solides, elles sont utilisées de façon classique en médecine humaine. Ces compositions peuvent être, par exemple, des solutions et des suspensions, injectables ou non et des comprimés, des capsules, des granulés, des gélules pour les formes solides.

Les exemples qui suivent illustrent de façon non limitative des formulations et des méthodes de fabrication de quelques compositions pharmaceutiques appropriées à l'administration des produits de l'invention.

| Comprimés | Quantité pour 100 g |
|---|---|
| Produit de l'exemple 1 | 0,50 g |
| Cellulose microcristalline | 93,50 g |
| Polyvinylpyrrolidone | 1,00 g |
| Carboxyméthylamidon | 4,00 g |
| Stéarate de magnésium | 1,00 g |

Technique de fabrication

Dans un mélangeur approprié, la cellulose microcristalline et une partie de carboxyméthylamidon sont mouillées avec une solution alcoolique de polyvinylpyrrolidone dans laquelle a été dissous le principe actif.

Le granulé obtenu est alors séché, calibré, puis additionné de stéarate de magnésium et du solde de carboxyméthylamidon, et enfin comprimé sur pastilleuse rotative.

| Pommade | Quantité pour 100 g |
|---|---|
| Produit de l'exemple 8 micronisé | 0,050 g |
| Vaseline | 40,000 g |
| Huile de vaseline | 15,000 g |
| Cire blanche | 4,000 g |
| Sesquioléate de sorbitan | 6,000 g |
| Eau purifiée q.s.p. | 100,000 g |

Technique de fabrication

La phase aqueuse chauffée à 75°C est incorporée sous agitation à la phase grasse préalablement chauffée à 70°C.

Le principe actif micronisé est incorporé dans la pommade.

Après homogénéisation, la pommade est répartie en tubes.

| Crème | Quantité pour 100 g |
|---|---|
| Produit de l'exemple 7 micronisé | 0,050 g |
| Alcool cétostéarylique | 8,000 g |
| Huile de vaseline | 10,000 g |
| Myristate d'isopropyle | 5,000 g |
| Propylène glycol | 5,000 g |
| Monostéarate de sorbitan POE | 2,500 g |
| Monostéarate de sorbitan | 1,500 g |
| Acide sorbique | 0,200 g |
| Hydroxyde de sodium q.s.pH | 5,5 |
| Eau purifiée q.s.p. | 100,000 g |

Technique de fabrication

L'acide sorbique est dissous dans la phase grasse préalablement chauffée à 70°C.

La phase aqueuse chauffée à 75°C et contenant les émulsionnants est ajoutée à la phase gazeuse.

Le principe actif micronisé est incorporé dans l'émulsion ainsi formée.

Après homogénéisation, la crème est répartie en tubes.

| Lavement | Quantité pour 100 ml |
|---|---|
| Produit de l'exemple 9 | 0,005 g |
| Polysorbate 80 | 0,050 g |
| Phosphate monosodique | 0,150 g |
| Clorure de sodium | 0,700 g |
| Carboxyméthylcellulose sodique à faible viscosité | 0,500 g |
| Alcool benzylique | 0,900 g |
| Hydroxyde de sodium q.s.p.pH | 6,8 |
| Eau purifiée q.s.p. | 100,000 ml |

Technique de fabrication

Dans l'eau purifiée sont dissous:
le polysorbate 80,
le phosphate monosodique,
le chlorure de sodium.

Dans cette solution sont ensuite dispersés:
la carboxyméthylcellulose sodique,
le produit actif micronisé

Le pH de la suspension est ajusté à 6,8 avec une solution d'hydroxyde de sodium.

Après homogénéisation, la suspension est répartie en flacons à raison de 100 ml par flacon.

| Suspension nasale et auriculaire | Quantité pour 100 ml |
|---|---|
| Produit de l'exemple 2 micronisé | 1,000 g |
| Chlorure de N-cétylpyridinium | 0,020 g |
| Phosphate monosodique | 0,150 g |
| Chlorure de sodium | 0,750 g |
| Hydroxyde de sodium q.s.p.pH | 6,8 |
| Alcool benzylique | 0,900 g |
| Eau purifiée q.s.p. | 100,000 ml |

Techniques de fabrication

Dans l'eau purifiée sont dissous sous agitation:
le chlorure de N-cétylpyridinium,
le phosphate monosodique,
le chlorure de sodium,
l'alcool benzylique.

Le principe actif micronisé est dispersé dans la solution précédente. Le pH de la suspension est ensuite ajusté à pH 6,8 avec une solution d'hydroxyde de sodium.

Après homogénéisation, la suspension est ensuite répartie à raison de 10 ml par flacon.

| Aérosol | Quantité pour un flacon de 10 ml |
|---|---|
| Produit de l'exemple 1 micronisé | 10 mg |
| Trioléate de sorbitan | 50 mg |
| Trichlorofluorométhane (F11) Dichlorodifluorométhane (F12) q.s.p. Dichlorotétrafluoroéthane (F114) | 10 ml |

Techniques de fabrication

Le principe actif est mis en suspension dans une partie du trichlorométhane (F11) dans lequel le trioléate de sorbitan a été préalablement dispersé.

. La suspension obtenue est répartie en flacons aérosols qui sont ensuite sertis avec une valve doseuse.

Le solde du trichlorofluorométhane ainsi que le dichlorodifluorométhane et dichlorotétrafluoroéthane sont alors injectés.

| Suspension injectable | Quantité pour 100 ml |
|---|---|
| Produit de l'exemple 4 micronisé | 2,000 g |
| Polysorbate 80 | 0,050 g |
| Chlorure de sodium | 0,780 g |
| Carboxyméthylcellulose sodique à faible viscosité | 0,500 g |
| Alcool benzylique | 0,900 g |
| Eau pour préparation injectable q.s.p. | 100,000 ml |

Technique de fabrication

Dans une partie de l'eau pour préparation injectable sont dissous:
le polysorbate 80,
le chlorure de sodium,
l'alcool benzylique.

La solution est alors stérilisée et, dans cette solution, est dispersé le principe actif micronisé lui-même préalablement stérilisé.

A la suspension est ajoutée une solution stérile de carboxyméthylcellulose sodique.

Après homogénéisation la suspension est répartie en ampoules à raison de 5 ml par ampoule.

| Lotion | Quantité pour 100 ml |
|---|---|
| Produit de l'exemple 7 micronisé | 0,100 g |
| Glycérine | 7,500 g |
| Alcool isopropylique | 15,000 g |
| Carboxypolyméthylène | 0,150 g |
| Hydroxyde de sodium q.s.p. pH | 7 |
| Eau purifiée q.s.p. | 100,000 ml |

Technique de fabrication

Le principe actif est dispersé dans le phase hydroalcoolique qui est ensuite légèrement gélifiée par addition de carboxypolyméthylène et d'hydroxyde de sodium.

Indications therapeutiques

Les produits de l'invention montrent une puissante activité anti-inflammatoire et ne présentent que de faibles effets glucocorticoïdes.

Les compositions pharmaceutiques contenant ces produits sont particulièrement utiles pour traiter des affections inflammatoires, prurigineuses et allergiques.

Au niveau de la peau et des muqueuses, elles trouvent leur utilisé dans le traitement des affections rectales et du colon, des eczémas et dermatites d'étiologies diverses, des lichénifications et du psoriasis. De même, leur action sur les muqueuses de la sphère O.R.L. et des bronches est particulièrement utile, notamment dans le traitement de l'asthme.

Ces compositions sont également utiles pour le traitement d'affections internes, comme les arthrites et les polyarthrites ainsi que pour les diverses maladies d'origines allergiques.

**Revendications pour les Etats contractants DE, LU, NL, SE**

1. Composés de formule:

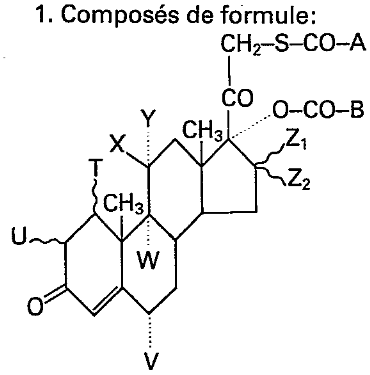

dans laquelle A et B sont chacun, indépendamment l'un de l'autre, un radical alcoyle linéaire ou ramifié ayant de 1 à 6 atomes de carbone ou un radical phényle éventuellement mono- ou plurisubstitué par des radicaux alcoyle ayant de 1 à 6 atomes de carbone, alcoxy ayant de 1 à 6 atomes de carbone ou halogène, T et U sont, indépendamment l'un de l'autre, des atomes d'hydrogène ou forment ensemble une double liaison, V est un atome d'hydrogène ou un radical méthyle en position α, W est un atome d'hydrogène ou un atome d'halogène en position α, X est un radical hydroxy en position β et Y est un atome d'hydrogène ou X et Y peuvent représenter ensemble un atome d'oxygène, et $Z_1$ est un atome d'hydrogène, un radical méthyle en position α ou β, alors que $Z_2$ est un atome d'hydrogène ou $Z_1$ et $Z_2$ forment ensemble un radical méthylène.

2. Le 21-thiopropionate 17-propionate de 3,20-dione-9α fluoro-11β hydroxy-16β méthyl-prégnadiène-1,4.

3. Le 21-thiovalérate 17-valérate de 3,20-dione-9α fluoro-11β hydroxy-16β méthyl-prégnadiène-1,4.

4. Le 21-thiotertiobutylate 17-acétate de 3,20-dione-9α fluoro-11β hydroxy-6α méthyl-prégnadiène-1,4.

5. Le 21-thiopropionate 17-propionate de 3,20-dione-9α chloro-11β hydroxy-16β méthyl-prégnadiène-1,4.

6. Composé suivant la revendication 1, qui sont:

a) le 21-thiovalérate,17-valérate de 3,20-dione-11β-hydroxy-prègn-4-ène;

b) le 21-thiopivalate,17-valérate de 3,20-dione-11β-hydroxy-prègn-4-ène;

c) le 21-thioacétate,17-butyrate de 3,11,20-trione prègna-diène-1,4;

d) le 21-thioacétate-17-benzoate de 3,20-dione-11β hydroxy-6α méthyl-prègna-diène-1,4;

e) le 21-thiopropionate-17-propionate de 3,20-dione-11β-hydroxy-6α-méthyl-prègna-diène-1,4;

f) le 21-thiovalérate-17-valérate de 3,20-dione-11β-hydroxy-6α-méthyl-prègna-diène-1,4;

g) le 21-thioacétate-17-valérate de 3,20-dione-11β-hydroxy-6α-méthyl-prègna-diène-1,4;

h) le 21-thioacétate,17-acétate de 3,20-dione-9α-fluoro-11β-hydroxy-16β-méthyl-prègna-diène-1,4;

i) le 21-thiopropionate 17-acétate de 3,20-dione-9α-fluoro-11β-hydroxy-16β-méthyl-prègna-diène;

j) le 21-thioacétate 17-propionate de 3,20-dione-9α-fluoro-11β-hydroxy-16β-méthyl-prègna-diène-1,4;

k) le 21-thiopropionate 17-propionate de 3,20-dione-11β-hydroxy-16-méthylène-prègna-diène-1,4;

l) le 21-thioacétate-17-valérate de 3,20-dione-9α-fluoro-11β-hydroxy-16β-méthyl-prègna-diène-1,4;

m) le 21-thiovalérate-17-propionate de 3,20-dione-9α-chloro-11β-hydroxy-16β-méthyl-prègna-diène-1,4;

n) le 21-thioacétate-17-valérate de 3,20-dione-9α-chloro-11β-hydroxy-16β-méthyl-prègnadiène-1,4;

o) le 21-thiovalérate-17-valérate de 3,20-dione-9α-chloro-11β-hydroxy-16β-méthyl-prègnadiène-1,4;

7. Procédé de préparation des composés suivant la revendication 1, caractérisé en ce qu'il consiste à faire réagir un 21-sulfonate de formule:

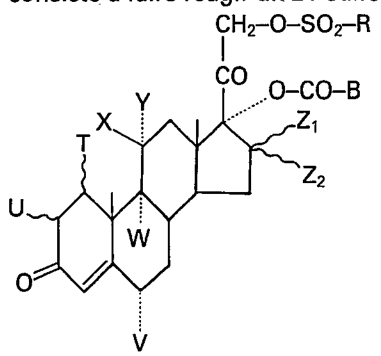

dans laquelle R est un radical alcoyle inférieur et les autres symboles ont la même signification qu'à la revendication 1, sur un thiocarboxylate de métal alcalin de formule:

M–S–CO–A

dans laquelle M est un atome de métal alcalin et A a la même signification qu'à la revendication 1.

8. Procédé suivant la revendication 7, caractérisé en ce qu'il consiste à effectuer la réaction dans un solvant aprotique, en engageant un défaut stoechiométrique du sulfonate en opérant entre 0 et 100°C environ pendant 5 min à 10 h environ.

9. Médicament, notamment anti-inflammatoire, anti-prurigineux, anti-allergique et anti-asthmatique, caractérisé en ce qu'il comprend un composé suivant l'une des revendications 1 à 6.

**Revendications pour l'Etat contractant AT**

1. Procédé de préparation des composés de formule:

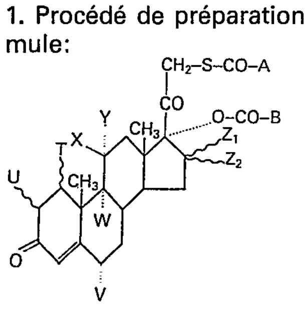

dans laquelle A et B sont chacun, indépendamment l'un de l'autre, un radical alcoyle linéaire ou ramifié ayant de 1 à 6 atomes de carbone ou un radical phényle éventuellement mono- ou pluri-substitué par des radicaux alcoyle ayant de 1 à 6 atomes de carbone, alcoxy ayant de 1 à 6 atomes de carbone ou halogène, T et U sont, indépendamment l'un de l'autre, des atomes d'hydrogène ou forment ensemble une double liaison, V est un atome d'hydrogène ou un radical méthyle en position α, W est un atome d'hydrogène ou un atome d'halogène en position α, X est un radical hydroxy en position β et Y est un atome d'hydrogène ou X et Y peuvent représenter ensemble un atome d'oxygène, et $Z_1$ est un atome d'hydrogène, un radical méthyle en position α ou β, alors que $Z_2$ est un atome d'hydrogène ou $Z_1$ et $Z_2$ forment ensemble un radical méthylène, caractérisé en ce qu'il consiste à faire réagir un 21-sulfonate de formule:

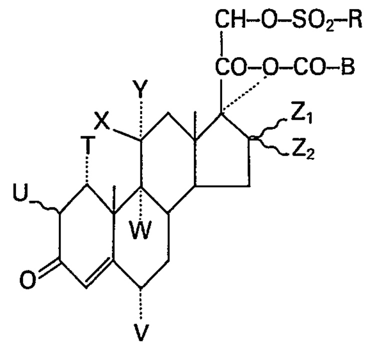

dans laquelle R est un radical alcoyle inférieur sur un thiocarboxylate de métal alcalin de formule:

M–S–CO–A

dans laquelle M est un atome de métal alcalin.

2. Procédé suivant la revendication 1, caractérisé en ce qu'il consiste à effectuer la réaction dans un solvant aprotique, en engageant un défaut stoechiométrique du sulfonate en opérant entre 0 et 100°C environ pendant 5 min à 10 h environ.

**Patentansprüche für die Vertragsstaaten DE, LU, NL und SE**

1. Verbindungen der nachstehenden allgemeinen Strukturformel

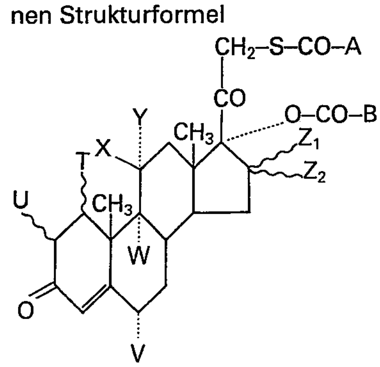

wobei A und B jeweils unabhängig voneinander für einen unverzweigten oder verzweigten $C_1$–$C_6$-Alkylrest oder einen Phenylrest stehen, der seiner-

seits gegebenenfalls einfach oder mehrfach mit einem oder mehreren $C_1$–$C_6$-Alkylrest(en), $C_1$–$C_6$-Alkoxyrest(en) oder Halogen substituiert sein kann;
T und U unabhängig voneinander für Wasserstoffatome stehen oder zusammen eine Doppelbindung bilden;
V für ein Wasserstoffatom oder einen Methylrest in α-Stellung steht;
W für ein Wasserstoffatom oder ein Halogenatom in α-Stellung steht;
X für ein Hydroxyrest in β-Stellung, und
Y für ein Wasserstoffatom oder X und Y gemeinsam für ein Sauerstoffatom stehen, und
$Z_1$ für ein Wasserstoffatom oder einen Methylrest in α- oder β-Stellung, und
$Z_2$ für ein Wasserstoffatom stehen oder $Z_1$ und $Z_2$ gemeinsam eine Methylengruppe bilden.

2. 9α-Fluoro-11β-hydroxy-16β-methyl-17-propionat-21-thiopropionat-pregna-1,4-dien-3,20-dion.

3. 9α-Fluoro-11β-hydroxy-16β-methyl-17-valerat-21-thiovalerat-pregna-1,4-dien-3,20-dion.

4. 9α-Fluoro-11β-hydroxy-6α-methyl-17-acetat-21-thiotertiobutylat-pregna-1,4-dien-3,20-dion.

5. 9α-Chloro-11β-hydroxy-16β-methyl-17-propionat-21-thiopropionat-pregna-1,4-dien-3,20-dion.

6. Verbindungen nach Anspruch 1, nämlich
a) 11β-Hydroxy-17-valerat-21-thiovalerat-pregn-4-en-3,20-dion;
b) 11β-Hydroxy-17-valerat-21-thiopivalat-pregn-4-en-3,20-dion;
c) 17-Butyrat-21-thioacetat-pregna-1,4-dien-3,11,20-trion;
d) 11β-Hydroxy-6α-methyl-17-benzoat-21-thioacetat-pregna-1,4-dien-3,20-dion;
e) 11β-Hydroxy-6α-methyl-17-propionat-21-thiopropionat-pregna-1,4-dien-3,20-dion;
f) 11β-Hydroxy-6α-methyl-17-valerat-21-thiovalerat-pregna-1,4-dien-3,20-dion;
g) 11β-Hydroxy-6α-methyl-17-valerat-21-thioacetat-pregna-1,4-dien-3,20-dion;
h) 9α-Fluoro-11β-hydroxy-16β-methyl-17-acetat-21-thioacetat-pregna-1,4-dien-3,20-dion;
i) 9α-Fluoro-11β-hydroxy-16β-methyl-17-acetat-21-thiopropionat-pregna-1,4-dien-3,20-dion;
j) 9α-Fluoro-11β-hydroxy-16β-methyl-17-propionat-21-thioacetat-pregna-1,4-dien-3,20-dion;
k) 11β-Hydroxy-16-methylen-17-propionat-21-thiopropionat-pregna-1,4-dien-3,20-dion;
l) 9α-Fluoro-11β-hydroxy-16β-methyl-17-valerat-21-thioacetat-pregna-1,4-dien-3,20-dion;
m) 9α-Chloro-11β-hydroxy-16β-methyl-17-propionat-21-thiovalerat-pregna-1,4-dien-3,20-dion;
n) 9α-Chloro-11β-hydroxy-16β-methyl-17-valerat-21-thioacetat-pregna-1,4-dien-3,20-dion;
o) 9α-Chloro-11β-hydroxy-16β-methyl-17-valerat-21-thiovalerat-pregna-1,4-dien-3,20-dion.

7. Verfahren zur Herstellung der Verbindung nach Anspruch 1, dadurch gekennzeichnet, dass ein 21-Sulfonat der nachstehenden allgemeinen Strukturformel

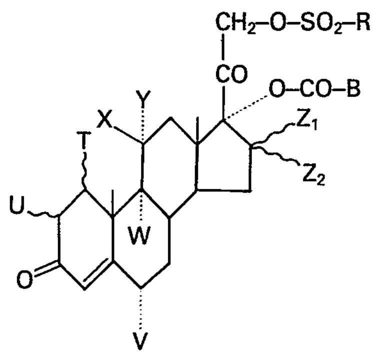

wobei B, T, U, V, W, X, Y, $Z_1$ und $Z_2$ die aus Anspruch 1 ersichtliche Bedeutung haben, und R für einen niederen Alkylrest steht, mit einem Alkalimetall-thiocarboxylat der nachstehenden allgemeinen Formel

M–S–CO–A

umgesetzt wird, wobei M für ein Alkalimetallatom steht, und A die in Anspruch 1 angegebene Bedeutung hat.

8. Verfahren nach Anspruch 7, dadurch gekennzeichnet, dass die Umsetzung in einem aprotischen Lösungsmittel 5 min bis 10 h lang zwischen 0 und 100°C durchgeführt, und das Sulfonat in einem unterstöchiometrischen Anteil eingesetzt wird.

9. Arzneimittel, insbesondere mit entzündungshemmender Wirkung, mit Juckreiz-beseitigender Wirkung, mit anti-allergischer Wirkung und/oder mit anti-asthmatischer Wirkung, dadurch gekennzeichnet, dass es als Wirkstoff eine Verbindung nach einem der Ansprüche 1 bis 6 enthält.

**Claims for the contracting states DE, LU, NL, SE**

1. Compounds of formula:

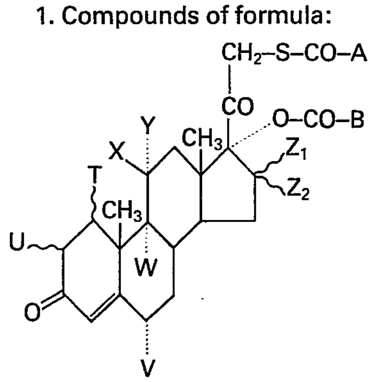

wherein A and B each represent, independently of each other, a straight-chained or branched alkyl group having from 1 to 6 carbon atoms or a phenyl group optionally mono- or polysubstituted by alkyl radicals having from 1 to 6 carbon atoms, alkoxy groups, having from 1 to 6 carbon atoms or halogen, T and U, independently of each other, represent hydrogen atoms or together from a double bond, V is a hydrogen atom or a methyl group at the α-position, W is a hydrogen atom or a halogen atom at the α-position, X is a hydroxy group at the β-position and Y is a hydrogen atom

or X and Y may together represent an oxygen atom, and $Z_1$ is a hydrogen atom, a methyl group at the α- or β-position, whilst $Z_2$ is a hydrogen atom, or $Z_1$ and $Z_2$ together form a methylene group.

2. 9α-Fluoro-11β-hydroxy-16β-methyl-pregna-1,4-diene-3,20-dione 17-propionate, 21-thiopropionate.

3. 9α-Fluoro-11β-hydroxy-16β-methyl-pregna-1,4-diene-3,20-dione 17-valerate 21-thiovalerate.

4. 9α-Fluoro-11β-hydroxy-16β-methyl-pregna-1,4-diene-3,20-dione 17-acetate, 21-thiotert-butylate.

5. 9α-Chloro-11β-hydroxy-16β-methyl-pregna-1,4-diene-3,20-dione 17-propionate, 21-thiopropionate.

6. The compounds of claim 1 which are:

a) 11β-Hydroxy-pregn-4-ene-3,20-dione 17-valerate, 21-thiovalerate.

b) 11β-Hydroxy-pregn-4-ene-3,20-dione 17-valerate, 21-thiopivalate.

c) Pregna-1,4-diene-3,11,20-trione 17-butyrate, 21-thioacetate.

d) 11β-Hydroxy-6α-methyl-pregna-1,4-diene-3,20-dione 17-benzoate, 21-thioacetate.

e) 11β-Hydroxy-6α-methyl-pregna-1,4-diene-3,20-dione 17-propionate, 21-thiopropionate.

f) 11β-Hydroxy-6α-methyl-pregna-1,4-diene-3,20-dione 17-valerate, 21-thiovalerate.

g) 11β-Hydroxy-6α-methyl-pregna-1,4-diene-3,20-dione 17-valerate, 21-thioacetate.

h) 9α-Fluoro-11β-hydroxy-16β-methyl-pregna-1,4-diene-3,20-dione 17-acetate, 21-thioacetate.

i) 9α-Fluoro-11β-hydroxy-16β-methyl-pregna-1,4-diene-3,20-dione 17-acetate, 21-thiopropionate.

j) 9α-Fluoro-11β-hydroxy-16β-methyl-pregna-1,4-diene-3,20-dione 17-propionate, 21-thioacetate.

k) 11β-Hydroxy-16-methylene-pregna-1,4-diene-3,20-dione 17-propionate, 21-thiopropionate.

l) 9α-Fluoro-11β-hydroxy-16β-methyl-pregna-1,4-diene-3,20-dione 17-valerate, 21-thioacetate.

m) 9α-Chloro-11β-hydroxy-16β-methyl-pregna-1,4-diene-3,20-dione 17-propionate, 21-thiovalerate.

n) 9α-Chloro-11β-hydroxy-16β-methyl-pregna-1,4-diene-3,20-dione 17-valerate, 21-thioacetate.

o) 9α-Chloro-11β-hydroxy-16β-methyl-pregna-1,4-diene-3,20-dione 17-valerate, 21-thiovalerate.

7. A process for the preparation of compounds of claim 1, characterized in that a 21-sulphonate of formula:

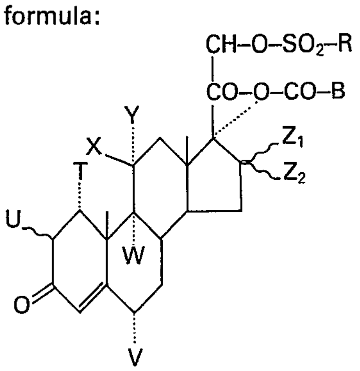

wherein R is a lower alkyl radical and the other symbols have the same meanings as in claim 1, is reacted with an alkali metal thiocarboxylate of formula:

M–S–CO–A

wherein M is an alkali metal atom and A has the same meaning as in claim 1.

8. The process of claim 7, characterized in that the reaction is carried out in an aprotic solvent with a stoichiometric deficiency of the sulphonate at from about 0 to 100°C for a period from about 5 min to 12 h.

9. An anti-inflammatory, anti-pruriginous, anti-allergic and anti-asthmatic medicament, characterized in that it comprises an active amount of a compound as claimed in anyone of claims 1 to 6.

**Patentansprüche für den Vertragsstaat AT**

1. Verfahren zur Herstellung von Verbindungen der nachstehenden allgemeinen Strukturformel

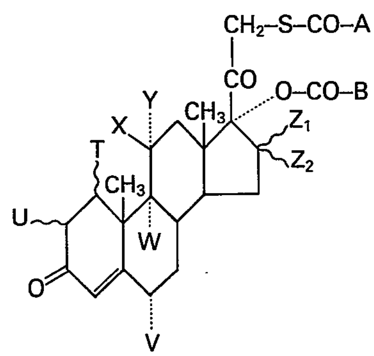

wobei A und B jeweils unabhängig voneinander für einen unverzweigten oder verzweigten $C_1$–$C_6$-Alkylrest oder einen Phenylrest stehen, der seinerseits gegebenenfalls einfach oder mehrfach mit einem oder mehreren $C_1$–$C_6$-Alkylrest(en), $C_1$–$C_6$-Alkoxyrest(en) oder Halogen substituiert sein kann;

T und U unabhängig voneinander für Wasserstoffatome stehen oder zusammen eine Doppelbindung bilden;

V für ein Wasserstoffatom oder einen Methylrest in α-Stellung steht;

W für ein Wasserstoffatom oder ein Halogenatom in α-Stellung steht;

X für einen Hydroxyrest in β-Stellung, und

Y für ein Wasserstoffatom oder X und Y gemeinsam für ein Sauerstoffatom stehen, und

$Z_1$ für ein Wasserstoffatom oder einen Methylrest in α- oder β-Stellung, und

$Z_2$ für ein Wasserstoffatom stehen oder $Z_1$ und $Z_2$ gemeinsam eine Methylengruppe bilden, dadurch gekennzeichnet, dass ein 21-Sulfonat der nachstehenden allgemeinen Strukturformel

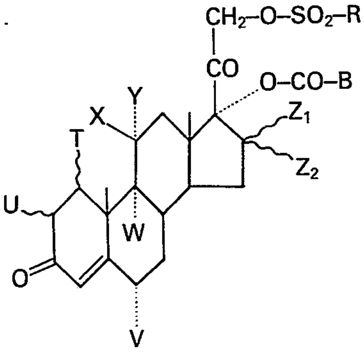

wobei R für einen niederen Alkylrest steht, mit einem Alkalimetall-thiocarboxylat der nachstehenden allgemeinen Formel

M–S–CO–A

umgesetzt wird, wobei M für ein Alkalimetallatom steht.

2. Verfahren nach Anspruch 1, dadurch gekennzeichnet, dass die Umsetzung in einem aprotischen Lösungsmittel 5 min bis 10 h lang zwischen 0 und 100°C durchgeführt, und das Sulfonat in einem unterstöchiometrischen Anteil eingesetzt wird.

**Claims for the contracting state AT**

1. Process for the preparation of compounds of formula:

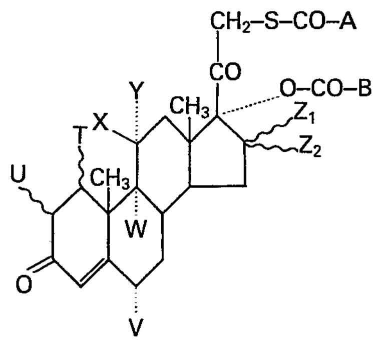

wherein A and B each represent, independently of each other, a straight-chained or branched alkyl group having from 1 to 6 carbon atoms or phenyl group optionally mono- or polysubstituted by alkyl radicals having from 1 to 6 carbon atoms, alkoxy groups having from 1 to 6 carbon atoms or halogen, T and U, independently of each other, represent hydrogen atoms or together form a double bond, V is a hydrogen atom or a methyl group at the α-position, W is a hydrogen atom or a halogen atom at the α-position, X is a hydroxy group at the β-position and Y is a hydrogen atom or X and Y may together represent an oxygen atom, and $Z_1$ is a hydrogen atom, a methyl group at the α- or β-position, whilst $Z_2$ is a hydrogen atom, or $Z_1$ and $Z_2$ together form a methylene group characterized in that a 21-sulphonate of formula

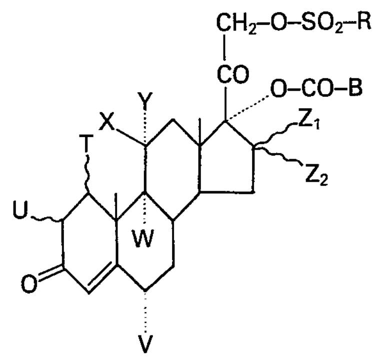

wherein R is a lower alkyl radical is reacted with an alkali metal thiocarboxylate of formula:

M–S–CO–A

wherein M is an alkali metal atom.

2. The process of claim 1, characterized in that the reaction is carried out in an aprotic solvent with a stoichiometric deficiency of the sulphonate at from about 0 to 100°C for a period from about 5 min to 12 h.